# EUROPÄISCHE PATENTSCHRIFT

(11) **EP 2 948 191 B1**
(45) Veröffentlichungstag und Bekanntmachung des Hinweises auf die Patenterteilung: **07.12.2016**
(21) Anmeldenummer: 13705111.6
(22) Anmeldetag: 25.01.2013
(51) Int. Cl.: B01D 69/10, B01D 71/70

(54) **RAUMLUFTFILTERSYSTEM**
ROOM AIR FILTER SYSTEM
SYSTÈME DE FILTRATION D'AIR AMBIANT

(43) Veröffentlichungstag der Anmeldung: 02.12.2015
(73) Patentinhaber: Hauser Umwelt-Service GmbH & Co. KG, 47809 Krefeld (DE)
(72) Erfinder: HAUSER, Hansgeorg, 47799 Krefeld (DE); PLÄNKER, Carsten, 46282 Dorsten (DE); BRANDES, Jörg, 47608 Geldern (DE); BUSCHMANN, Hans-Jürgen, 47906 Kempen (DE)
(74) Vertreter: Schneiders, Josef
(86) Internationale Anmeldenummer: PCT/EP2013/051416
(87) Internationale Veröffentlichungsnummer: WO 2014/114345

(56) Entgegenhaltungen:
- WO-A2-2006/049680
- DE-A1-102006 001 528
- DE-A1-102006 010 561
- JP-A- 2004 089 982
- DATABASE INSPEC [Online] THE INSTITUTION OF ELECTRICAL ENGINEERS, STEVENAGE, GB; Juli 2007 (2007-07), LI PI-GAO ET AL: "Application of [beta]-cyclodextrin-triacetin suspension in cigarette filter", XP002712840, Database accession no. 10077161 & TOBACCO SCIENCE & TECHNOLOGY, Nr. 7, Juli 2007 (2007-07), Seiten 5-7 , 26, EDITORIAL OFFICE OF CHINESE JOURNAL OF TOBACCO SCIENCE AND TECHNOLOGY CHINA ISSN: 1002-0861

## Beschreibung

Die Erfindung betrifft ein Raumluftfiltersystem zum Ausfiltern von gasförmigen Geruchs- und Schadstoffen mit einer sorptiv wirkenden ersten Filtereinheit und einer schadstoffbindenden zweiten Filtereinheit.

Raumluftfiltersystems mit verschiedenen Filtermaterialien sind seit Langem zur Verbesserung der Luftqualität im Einsatz. Eingesetzt werden sie beispielsweise zur Geruchsbindung, zur Bindung von Schadstoffen, zur Ausfilterung von Keimen, aber auch zur Beseitigung von Staub und Allergenen. Neben sorptiv wirkenden Filtermaterialien, etwa Aktivkohle, werden auch Flüssigkeiten zur Adsorption von Schadstoffen wie auch Elektrofilter eingesetzt. Ein weiteres Filterprinzip beruht auf der Abscheidung von festen Schmutzpartikeln an aus Fasern hergestellten Vliesmedien. Ferner kommen auch Flüssigkeiten, durch die die zu reinigende Luft geführt wird und in denen die gasförmigen Schadstoffe gelöst zurückbleiben zum Einsatz.

Zu unterscheiden ist zwischen festen Schadstoffen und in der Raumluft verteilten gasförmigen Schadstoffen. Während erstere häufig durch einfache mechanische Mittel abgetrennt werden können, sind bei letzteren sorptiv wirkende Systeme notwendig.

Ein Problem sorptiv wirkender Filtersysteme ist häufig technisch bedingt und beruht auf deren spezifischen Sorptionscharakteristiken.

Zum Einen stellt sich zwischen dem Sorptionsmaterial und der Umgebungsluft ein Gleichgewicht ein, d. h. im gleichen Maße wie Schadstoff vom Sorptionsmaterial aufgenommen wird, wird ein (geringer) Teil auch wieder abgegeben. Der das Sorptionsmaterial passierende Luftstrom ist nur schadstoffabgereichert. Dies gilt insbesondere auch für Aktivkohle, die regelmäßig als Absorptionsmittel eingesetzt wird.

Zum Anderen werden von vielen Sorptionsmaterialien Schadstoffe aufgrund geringer Affinität nur zu einem Teil überhaupt aufgenommen werden. Ein Teil der Schadstoffe passiert das Filtersystem und verbleibt in der nur partiell gereinigten Raumluft.

Hinzu kommt das Problem der Sättigung des Filtermaterials gegen Ende seiner Aufnahmekapazität oder Lebensdauer. In diesem Fall verbleibt zunehmend Schadstoff in der zu filternden Raumluft.

Wünschenswert ist aber eine möglichst hohe Abscheidungsrate der Schadstoffe aus der zu filternden Luft.

Ein häufiges Einsatzgebiet von Raumluftfiltern ist die Reinigung von rauchgeschwängerter Luft in Raucherlounges, Hotelzimmern, Bars, Restaurants, Spielhallen und anderen Einrichtungen dieser Art. Dies gilt insbesondere für Einrichtungen, in denen nicht permanent geraucht wird und denen sich Rauchrückstände in Textilien, Möbeln und an Wänden abgelagert haben. Diese werden über die Zeit erneut an die Raumluft abgegeben; die Benutzer solcher Räume empfinden die Luft dann als abgestanden und muffig.

Ein Problem sind dabei insbesondere auch Einrichtungen, in denen nach Eintritt gesetzlicher Rauchverbote die "alte" mit Rauchrückständen behaftete Einrichtung der weiteren Nutzung entgegensteht. Insbesondere mit Rauchrückständen gesättigte Teppichböden geben die darin gesammelten Rückstände über lange Zeit intensiv an die Raumluft ab. Neben Verbrennungsrückständen sind dies häufig auch toxische chemische Verbindungen, wie Nikotin, Pyridin, Phenol, Furane, Furfural und Triacetin.

Nikotin kann in sehr großen Mengen in Textilien gespeichert sein. Es ist hoch toxisch.

Weitere Einsatzgebiete für Raumluftfiltersysteme sind Orte, in denen es zu unerwünschter Geruchsbildung kommt, insbesondere auch in Klinken und ärztlichen Praxen (Desinfektionsmittel), in Toilettenanlagen, bei der Tierhaltung, im Umfeld von Tankstellen, Anlagen zur Erzeugung von chemischer Produkte, in Chemikalienhandlungen und dergleichen. Ferner kommen Raumluftfiltersysteme in öffentlichen Einrichtungen mit hoher Publikumsfrequenz, bei der Entgiftung schadstoffbelasteter Räume, in Schulen, Alten- und Pflegeheimen sowie in Museen zum Einsatz.

DE102006010561 offenbart eine biozide oder biostatische und geruchsabsorbierende Ausrüstung eines organischen polymeren und/oder anorganischen Materials und der hieraus erhaltenen Erzeugnisse, enthaltend 0,1 Gew.-% bis 10 Gew.-% adsorptiv an der Oberfläche haftende Cyclodextrine oder Cyclodextrinderivate, die ausgewählt sind aus der Gruppe, enthaltend alpha-, beta- und gamma-Cyclodextrin sowie Cyclodextrinderivate, wie vorzugsweise teilmethylierte Cyclodextrine und Hydroxypropylcyclodextrine. DE102006001528 und XP002712840 (Li Pi-Gao; Wang Zong-Ying; Li Gang; Li Xiao-Bin, Yan'an Univ., Coll. of Chem. & Chem. Eng., Yan'an, 716000,China, Tobacco Science & Technology, July 2007) lehren ein textiles Filtermaterial mit wenigstens einer textilen Trägerschicht und wenigstens einer Aktivkohle enthaltenden Schicht, das zusätzlich zu Cyclodextrin ein weiteres Adsorbens enthält, sowie eine damit ausgestattete Schutzkleidung.

Aufgabe der Erfindung ist es, ein Raumluftfiltersystem bereitzustellen, dass die Raumluft zuverlässig von insbesondere gasförmigen Schadstoffen befreit. Es soll insbesondere die Reinigungskapazität herkömmlicher sorptiv wirkender Filtersysteme erhöhen.

Diese Aufgabe wird mit einem Raumluftfiltersystem gemäß Anspruch 1 gelöst.

Unter erster Filtereinheit wird erfindungsgemäß auch eine Kombination aus zwei oder mehr sorptiven Filtern verstanden. Entsprechend kann eine zweite Filtereinheit zwei oder mehr bindende Filter enthalten. Die sorptiven und bindenden Filtermaterialien können dabei jeweils verschieden sein.

Bevorzugte chemische Verbindungen, die geeignet sind, Geruchs- und Schadstoffe in einem Hohlraum zubinden, sind Cyclodextrine, Cucurbiturile, und Calixarene und andere supramolekulare Komplexbildner, die sich aus einer definierten Anzahl von Monomereinheiten zusammensetzen. Es handelt sich dabei um makrozyklische Moleküle, die sich aus mehreren Einheiten zusammensetzen und ein inerten Hohlraum aufweisen, in den sie Fremdsubstanzen aufnehmen und festhalten können. Diese Verbindungen bilden definierte Komplexe aus, die die eingelagerten Verbindungen nur unter besonderen Bedingungen wieder abgeben. Bevorzugte chemische Verbindungen mit Hohlräumen, wie sie in der zweiten Filtereinheit zum Einsatz kommen, sind Cyclodextrine.

Cyclodextrine sind makrozyklische Moleküle, die aus sechs, sieben oder acht α-D-Glukoseeinheiten aufgebaut sind und einen definierten ringförmigen Hohlraum ausbilden. Cyclodextrine sind in der Lage, in diesen Hohlraum eine Vielzahl organischer Moleküle einzulagern. Sie werden im Bereich der Pharmazie, Kosmetik und Textiltechnik industriell eingesetzt. Einmal eingelagerte Moleküle sind dort quasi chemisch gebunden, ohne dass es aber zur Ausbildung von chemischen Bindungen kommt; sie werden nur dann erneut abgegeben, wenn sie durch andere Moleküle verdrängt werden, die eine stabilere Bindung "erfahren", etwa durch Wasser. Cydodextrine (wie auch Cucurbiturile und Calixarene) bilden mit den gelagerten Molekülen Komplexe mit definierter Stöchiometrie.

Unterschieden wird zwischen α-, β- und γ-Cyclodextrinen, die sich durch die Zahl der Glukoseeinheiten unterscheiden. Zumeist kommt β-Cyclodextrin zum Einsatz, dessen Hohlraum einen Durchmesser von etwa 650 pm aufweist. Cyclodextrine sind eingeschränkt von Wasser löslich und können aus wässeriger Lösung appliziert werden.

Erfindungsgemäß werden unter Cyclodextrinen α-, β- und γ-Cyclodextrine verstanden, wobei β-Cyclodextrin bevorzugt ist. Erfindungsgemäße Cyclodextrine sind auch die üblichen Derivate von α-, β- und γ-Cyclodextrinen, beispielsweise Methyl-substituierte Produkte. Für den Einsatz in Filtermedien müssen diese Cyclodextrine noch zusätzlich derivatisiert werden, um sie chemisch, physikalisch oder mechanisch an ein Trägermaterial zu binden. Diese Techniken sind bekannt und vielfach beschrieben.

Die erste wie die zweite Filtereinheit können in ihrer ersten und/oder zweiten Filtereinheit ein Trägermaterial aufweisen, das mit dem entsprechenden sorptiven oder bindenden Filtermaterialien versetzt ist.

Erfindungsgemäß wird unter Sorption die physikalische oder chemische Bindung von Schadstoffen verstanden, beispielsweise die Adsorption eines Schadstoffs in den Hohlräumen eines sorptiv wirkenden Stoffes, wie Aktivkohle, die Adsorption eines Schadstoffes auf der Oberfläche eines dazu geeigneten Stoffes wie auch die Chemiesorption eines Geruchstoffs auf einer dazu geeigneten Matrix. In der Regel kommt für die sorptiv wirkende erste Filtereinheit Aktivkohle in Frage. Es können aber auch andere Filtermaterialien verwandt werden, etwa Zeolithe (Molekularsiebe), Bentonite, Nanoclays und metallorganische Gerüste (MOFs).

Für die zweite Filtereinheit werden vorzugsweise Cyclodextrine auf einem Trägermaterial eingesetzt. Als Trägermaterialien kommen insbesondere textile Materialien in Frage, wobei diese vorzugsweise als Vliese vorliegen. Für die Verankerung der Cyclodextrine auf dem Trägermaterial können chemische Ankergruppen, wie z. B. Monochlorotriazinyl (MCT) (DE 44 29 229), Vinylsulfongruppen (EP 0 115 705) oder 2,3-Dibromopropionylgruppen (DE 101 55 781), die eine kovalente Bindung an cellulosische Materialien bewirken. Eine Fixierung auf Polyestermaterialien kann durch Alkylgruppen entsprechend substituierter Cyclodextrine erfolgen. Bei vielen Materialien reicht es in aller Regel aus, das Trägermaterial z. B. mit einem Polyvinylamin oder Polyethylenimin substituiertem Cyclodextrin (DE 10 2006 010 561) zu imprägnieren. Es wird durch elektrostatische Wechselwirkungen zwischen den Polymeroberflächen und den positiv geladenen Aminogruppen festgehalten. Da die Filtermaterialien nur einmal zum Einsatz kommen, besteht keine Gefahr, dass das Cyclodextrin sich davon trennt bzw. davon ausgewaschen wird. Die Imprägnierung ist Luftstrom stabil.

Cucurbiturile und Calixarene können zusammen mit einem Trägermaterial, aber auch als Schüttung eingesetzt werden.

Der Vorteil der Cyclodextrine in Kombination mit einem Aktivkohlefilter als erster Filtereinheit liegt darin, dass die geringen Mengen an Geruchs- und Schadstoffen, die die erste Filtereinheit passieren oder von dieser an den Luftstrom abgegeben werden, zuverlässig darin gebunden werden. Damit wird ein hoher Reinigungsgrad erhalten. Zudem dient die zweite Filtereinheit als Sicherheitsstufe, die bei Sättigung der ersten Filtereinheit für eine gewisse Zeit ihre Funktion übernehmen kann. In der Regel reicht aber die Kapazität der zweiten Filtereinheit nicht aus, die erste Filtereinheit über längere Zeit zu ersetzen.

In diesem Zusammenhang ist wichtig, dass die zweite Filtereinheit mit der höheren Bindungswirkung für Geruchs- und Schadstoffe der ersten Filtereinheit nachgeschaltet ist.

Als Trägermaterialien kommen eine Vielzahl unterschiedlicher Fasermaterialien in Frage, die synthetischer oder natürlicher Natur sein können. Dies können beispielsweise Baumwolle, Polyester, Polyamid, aber auch Glasfasern sein. Das Trägermaterial kann als Gewebe vorliegen, wird jedoch vorzugsweise als loses Vlies eingesetzt. Die Beladung mit den Cyclodextrinen erfolgt beispielsweise durch Imprägnierung aus einem wässrigen System.

Es ist bekannt, dass mit aminhaltigen Substituenten Cyclodextrine durch elektrostatische Wechselwirkungen an Oberflächen haften. Geeignet ist eine sehr große Anzahl von Aminverbindungen, von denen insbesondere Polyvinylamine, Polyethylenamine, Aminosiloxan, Aminocellulosederivate, Chitosan, Aminosäuren und Proteine in Frage kommen, auch Alkylamine.

Selbstverständlich kommt auch eine chemische Anbindung der Cyclodextrine an das Trägermaterial in Frage, beispielsweise über reaktiven Ankergruppen auf Basis von Isocyanaten. In Frage kommen hierfür ferner halogentriacinylsubstituierte Cyclodextrine.

Zur Bindung der Cyclodextrine kann das Trägermaterial beispielsweise mit 1 bis 10 Gew.-%, bezogen auf das Trägermaterial, mit Polyvinylamin-substituierten Cylodextrinen oder Chitosan imprägniert sein. Mit der wässerigen Lösung des Polyvinylamins und Chitosans kann gleichzeitig Cyclodextrin in einer Menge von 0,5 bis 5 Gew.-% aufgebracht werden, ebenfalls bezogen auf das Trägermaterial. Um eine hohe Beladung des Cyclodextrins zu erreichen, kann der Imprägnierschritt mehrfach vorgenommen werden. Das imprägnierte Material wird getrocknet und ist danach einsatzfähig.

Die erste Filtereinheit enthält das sorptive Filtermaterial, etwa Aktivkohle, vorzugsweise als Schüttung, aber auch in Form von Aktivkohlematten, als Aktivkohle in einem textilen oder polymeren Matrix oder als aktivkohlehaltiger Schaum.

Zur Einbringung der Filtermaterialien der ersten und zweiten Filtereinheit in ein Raumluftfiltersystem ist es zweckmäßig, diese Filtereinheiten als Einschübe oder Kartuschen vorzusehen, die in eine dafür vorgesehene Halterung eingepasst oder eingeschoben werden. Dies ermöglicht eine rasche Beladung des Filtersystems mit den aktiven Materialien und einen ebenso raschen Austausch. Nach vollständiger Beladung der Filtereinheiten können diese entsorgt werden, etwa durch Verbrennung in einer herkömmlichen Müllverbrennungsanlage.

Es kann sinnvoll sein, dass erfindungsgemäße Raumluftfiltersystem mit einem Partikelfilter auszustatten, der feste Partikel mechanisch aus der Raumluft ausfiltert. Dieser Partikel- oder Grobfilter sollte der ersten Filtereinheit vorgeschaltet sein. Die Raumluft wird mit Hilfe eines üblichen Gebläses durch die Filtereinheiten geführt.

Das erfindungsgemäße Raumluftfiltersystem hat - insbesondere bei Verwendung aktiver Aminverbindungen zur Fixierung des Cyclodextrins - auch biostatische Eigenschaften. Polyvinylamin hat sich für viele Mikroorganismen als biozid erwiesen, weshalb das Raumluftfiltersystem geeignet ist, die Keimzahl in der gefilterten Luft herabzusetzen. Viele Mikroorganismen werden auf dem mit Polyvinylamin ausgerüsteten Filter abgetötet.

Die erfindungsgemäßen Raumluftfiltersysteme eignen sich insbesondere zur Reinigung von Raumluft von Geruchsstoffen, wie sie beispielsweise in Zigarettenrauch auftreten. Ein weiteres Einsatzfeld ist die Reinigung von Räumen von aus Ablagerungen abgegebenen Schadstoffen, etwa aus Tabakwaren, insbesondere Nikotin und flüchtigen Teerprodukten. Eingesetzt werden kann es ferner in Toilettenanlagen, in Räumen, in denen Tiere gehalten werden, in Kliniken und Arztpraxen, die mit chemischen Desinfektionsmitteln beaufschlagt werden sowie in Räumen, die technisch oder tätigkeitsbedingt mit Chemikalien oder Brennstoffen wie Benzin, aber auch Reinigungsmitteln beaufschlagt werden.

Ein weiteres Einsatzfeld sind öffentliche Einrichtungen mit hoher Publikumsfrequenz, Alten- und Pflegeheime, Versammlungsräume, Hotels, Restaurants und dergleichen. Ein besonderes Einsatzfeld sind Museen, insbesondere deren Magazine, in denen häufig konservierende Substanzen wie Formaldehyd und Naphthalin freigesetzt werden.

Ein häufiges Problem sind mit Formaldehyd "verseuchte" Räume, die auf diese Art und Weise ebenfalls gereinigt werden können. Formaldehyd wird häufig zur Konservierung von Importprodukten, die aus tropischen Ländern stammen, verwand. Von dort bezogene Teppiche, Möbel und sonstige Einrichtungsgegenstände, aber auch Kleidung ist deshalb häufig mit hohen Konzentrationen von Formaldehyd , aber auch Fungiziden, Pestiziden und Herbiziden versehen, die über die Zeit ausgasen. Die Formaldehydkonzentration in derartigen Räumen kann erfindungsgemäß erheblich gesenkt werden.

Die nachfolgenden Beispiele sollen die Erfindung erläutern.

### Beispiel 1:

10 g Polyestervlies werden mit 1 g Polyvinylaminlösung gleichmäßig besprüht und an der Luft getrocknet. Dann wird die Probe mit einer 5 %igen Lösung von Monochlortrianzin-β-Cyclodextrin (CavatexW7MCT) gleichmäßig besprüht, bis sich im Vlies 2 bis 3 % des β-Cyclodextrins befinden. Anschließend wird das Vlies bei ca. 80°C getrocknet. Der Nachweis der elektrostatischen Bindung des Cyclodextrins an dem Vlies kann mit Hilfe einer basischen Lösung von Phenophthalien erfolgen, die durch das Cyclodextrin entfärbt wird.

Alternativ kann eine Lösung des Gyclodextrinderivats mit dem Polyvinylaminsubstituenten auf Vlies aufgesprüht werden. Die (wässrige) Sprühlösung sollte 2 bis 5 Gew.-% des Cyclodextrinderivats enthalten

### Beispiel 2:

Ein Raumluftfiltersystem wurde mit einem Grobfilter, einer ersten Filterschicht auf Basis von Aktivkohle und einer zweiten Filterschicht auf Basis von β-Cyclodextrin (siehe Beispiel 1) ausgestattet. Dieses Raumluftfiltersystem wurde über einen Zeitraum mit einer hohen Belastung durch Raucher eingesetzt. Nach vier Monaten wurden die Filtereinheiten auf die Gegenwart bestimmter typischer chemischer Verbindungen aus Zigarettenrauch hin analysiert. Es wurden die folgenden Werte ermittelt:
1. Grobfilter

| Substanz | Gehalt [mg/kg] | Bestimmungsgrenze |
|---|---|---|
| Nikotin | 3500 | 10 |
| Pyridin | u.B. | 10 |
| Isopren | u.B. | 10 |
| 2,5-Dimethylfuran | u.B. | 10 |
| Phenol | u.B. | 10 |
| o-Kresol | u.B. | 10 |
| m/p-Kresol | u.B. | 10 |
| Furfural | u.B. | 10 |
| Triacetin | u.B. | 10 |

| | | |
|---|---|---|
| u.B.: Messwert liegt unter der Bestimmungsgrenze der Methode | | |

1. Filterschicht

| Substanz | Gehalt [mg/kg] | Bestimmungsgrenze |
|---|---|---|
| Nikotin | 15000 | 10 |
| Pyridin | 70 | 10 |
| Isopren | 30 | 10 |
| 2,5-Dimethylfuran | 14 | 10 |
| Phenol | 200 | 10 |
| o-Kresol | 20 | 10 |
| m/p-Kresol | 70 | 10 |
| Furfural | 150 | 10 |
| Triacetin | 340 | 10 |

2. Filterschicht

| Substanz | Gehalt [mg/kg] | Bestimmungsgrenze |
|---|---|---|
| Nikotin | 300 | 10 |
| Pyridin | 120 | 10 |
| Isopren | 220 | 10 |
| 2,5-Dimethylfuran | 150 | 10 |
| Phenol | 23 | 10 |
| o-Kresol | 3 | 10 |
| m/p-Kresol | u.B. | 10 |
| Furfural | 200 | 10 |
| Triacetin | u.B. | 10 |

Das erfindungsgemäße Raumluftfiltersystem ist in der Lage, insbesondere Nikotin aus der Raumluft zu extrahieren. Die innerhalb von vier Monaten ermittelten Werte erreichten in der ersten Filtereinheit (Aktivkohle) 15.000 mg/kg Filtermasse. Eine Reihe weiterer typischer Substanzen aus Zigarettenrauch wurden ebenfalls gebunden.

Die zweite Filterschicht war ebenfalls, teilweise in erhöhtem Maße, mit Geruchs-und Schadstoffen beladen. Es handelt sich dabei um die Restmengen, die von dem Grobfilter bzw. dem der ersten Filtereinheit nicht adsorbiert wurden. Der Cyclodextrinfilter hat also seine Sicherheitsfunktion bezüglich der Beseitigung von Restmengen an Schadstoffen voll erfüllt. Seine Kapazität war nach mehr als vier Monaten noch nicht erschöpft.

### Beispiel 3:

In einer Reihe von orientierenden Versuchen wurde ermittelt, in welchem Umfang Keime und einige spezielle Substanzen mit erfindungsgemäßem Raumluftfiltersystem (RLF-Gerät) gebunden werden. Es zeigte sich dabei, dass beispielsweise Schimmelpilze, Formaldehyd, Toluol, Essigsäure, Butylacetat und Ammoniak in erheblichen Umfang aus der Raumluft gebunden werden. Eine Tabelle mit den Messergebnissen in einer Prüfkammer folgt nach.

**Koloniebildende Einheiten (KBE)**

| Substanz | Konzentration ohne RLF-Gerät [KBE/m³] | Konzentration mit RLF-Gerät [KBE/m³] |
|---|---|---|
| KBE / Schimmelpilze | 8452 | 632 |
| KBE / Schimmelpilze | 7863 | 451 |
| KBE / Schimmelpilze | 2530 | 398 |
| KBE / Schimmelpilze | 1841 | 412 |

| Substanz | Konzentration ohne RLF-Gerät [mg/m³] | Konzentration mit RLF-Gerät [mg/m³] |
|---|---|---|
| Formaldehyd | 0,28 | 0,07 |
| Formaldehyd | 0,31 | 0,05 |

**Lösungsmittel**

| Substanz | Konzentration ohne RLF-Gerät [µg/m³] | Konzentration mit RLF-Gerät [µg/m³] |
|---|---|---|
| Toluol | 44 | 8 |
| Essigsäure | 446 | 21 |
| Butylacetat | 384 | 6 |

**Ammoniak**

| Substanz | Konzentration ohne RLF-Gerät [ppm] | Konzentration mit RLF-Gerät [ppm] |
|---|---|---|
| Ammoniak | 71 | 4 |
| Ammoniak | 26 | 4 |
| Ammoniak | 12 | 4 |

**Nikotin**

| Substanz | Konzentration ohne RLF-Gerät [µg/m³] | Konzentration mit RLF-Gerät [µg/m³] |
|---|---|---|
| Nikotin | 36 | 4 |

## Patentansprüche

1. Raumluftfiltersystem zur Ausfilterung von gasförmigen Geruchs-und Schadstoffen mit einem Gebläse, einer sorptiv wirkenden 1. Filtereinheit und einer geruchs- und schadstoffbinden 2. Filtereinheit, wobei Raumluft mithilfe des Gebläse durch die Filtereinheiten geführt wird und die 2. Filtereinheit der 1. nachgeschaltet ist, wobei die Filtereinheiten als Einschübe oder Kartuschen ausgebildet sind, die 2. Filtereinheit eine chemische Verbindung enthält, die zur Bindung der Geruchs und Schadstoffe in einem Hohlraum befähigt ist, ausgewählt aus Cyclodextrinen, Cucurbiturilen und Calixarenen, und die 2. Filtereinheit ein Vlies als Trägermaterial aufweist das mit einem Amingruppen und der chemischen Verbindung enthaltenden Mittel imprägniert ist.

2. Raumluftfiltersystem nach Anspruch 1, **dadurch gekennzeichnet, dass** die chemische Verbindung ein Cyclodextrin ist.

3. Raumluftfiltersystem nach Anspruch 1 oder 2, **dadurch gekennzeichnet, dass** das Cyclodextrin β-Cyclodextrin ist.

4. Raumluft für das System nach einem der vorstehenden Ansprüche, **dadurch gekennzeichnet, dass** das Mittel ein Polyvinylamin enthält, das Cyclodextrin gebunden enthält.

5. Raumluft für das System nach Anspruch 4, **dadurch gekennzeichnet, dass** das Trägermaterial 1 bis 10 Gew.-%, bezogen auf das Gewicht des Trägermaterials, Polyvinylamin enthält.

6. Raumluftfiltersystem nach einem der Ansprüche 1 bis 5, **dadurch gekennzeichnet, dass** das Trägermaterial 0, 5 bis 5 Gew.-%, bezogen auf das Gewicht des Trägermaterials, Cyclodextrin enthält.

7. Raumluftfiltersystem nach einem der vorstehenden Ansprüche, **dadurch gekennzeichnet, dass** das 1. Filtersystem Aktivkohle enthält.

8. Raumluftfiltersystem, nach einem der vorstehenden Ansprüche, **dadurch gekennzeichnet, dass** der 1. Filtereinheit ein Partikelfilter vorgeschaltet ist.

9. Verwendung eines Raumluftfiltersystems nach einem der Ansprüche 1 bis 8 zur Reinigung von Raumluft von gasförmigen Geruchs- und Schadstoffen.

10. Verwendung nach Anspruch 9 zur Reinigung von Raumluft von Nikotin und anderen Produkten aus der Verbrennung/Verschwelung von Tabakwaren.

## Claims

1. Room air filter system for filtering out gaseous odorant and noxiant molecules with a blower, a sorptively acting 1st filtering unit and an odorant-and noxiant-binding 2nd filtering unit, wherein room air is ducted by the blower through the filtering units and the 2nd filtering unit is downstream of the 1st filtering unit, wherein the filtering units are constructed as inserts or cartridges, the 2nd filtering unit contains a chemical compound capable of binding odorant and noxiant molecules in a cavity selected from cyclodextrins, cucurbiturils and calixarenes, and the 2nd filtering unit includes a carrier material in the form of a fibrous nonwoven web impregnated with an agent containing amino groups and the chemical compound.

2. Room air filter system according to Claim 1, **characterized in that** the chemical compound is a cyclodextrin.

3. Room air filter system according to Claim 1 or 2, **characterized in that** the cyclodextrin is β-cyclodextrin.

4. Room air for the system according to any preceding claim, **characterized in that** the agent contains a polyvinylamine which contains cyclodextrin in bound form.

5. Room air for the system according to Claim 4, **characterized in that** the carrier material contains from 1 to 10 wt%, based on the weight of the carrier material, of polyvinylamine.

6. Room air filter system according to any of Claims 1 to 5, **characterized in that** the carrier material contains from 0.5 to 5 wt%, based on the weight of the carrier material, of cyclodextrin.

7. Room air filter system according to any preceding claim, **characterized in that** the 1st filtering system contains activated carbon.

8. Room air filter system according to any preceding claim, **characterized in that** the 1st filtering unit is preceded by a particle filter.

9. Use of a room air filter system according to any of Claims 1 to 8 for cleaning room air of gaseous odorant and noxiant molecules.

10. Use according to Claim 9 for cleaning room air of nicotine and other products from the burning/smouldering of tobacco goods.

## Revendications

1. Système de filtration de l'air ambient pour l'élimination par filtration de substances gazeuses odorantes et nocives avec un ventilateur, une 1^{ère} unité de filtration à effet de sorption et une 2^{ère} unité de filtration à fixation des substances odorantes et nocives, l'air ambiant étant conduit au travers des unités de filtration à l'aide du ventilateur et la 2^{ème} unité de filtration étant connectée en aval de la 1^{ère}, les unités de filtration étant configurées sous la forme de tiroirs ou de cartouches, la 2^{ème} unité de filtration contenant un composé chimique qui est apte à la fixation des substances odorantes et nocives dans une cavité, choisi parmi les cyclodextrines, les cucurbituriles et les calixarènes, et la 2^{ème} unité de filtration comprenant un non-tissé en tant que matériau support, qui est imprégné avec un agent contenant des groupes amine et le composé chimique.

2. Système de filtration de l'air ambiant selon la revendication 1, **caractérisé en ce que** le composé chimique est une cyclodextrine.

3. Système de filtration de l'air ambiant selon la revendication 1 ou 2, **caractérisé en ce que** la cyclodextrine est la β-cyclodextrine.

4. Air ambiant pour le système selon l'une quelconque des revendications précédentes, **caractérisé en ce que** l'agent contient une polyvinylamine qui contient une cyclodextrine reliée.

5. Air ambiant pour le système selon la revendication 4, **caractérisé en ce que** le matériau support contient 1 à 10 % en poids, par rapport au poids du matériau support, de polyvinylamine.

6. Système de filtration de l'air ambiant selon l'une quelconque des revendications 1 à 5, **caractérisé en ce que** le matériau support contient 0,5 à 5 % en poids, par rapport au poids du matériau support, de cyclodextrine.

7. Système de filtration de l'air ambiant selon l'une quelconque des revendications précédentes, **caractérisé en ce que** le 1^{er} système de filtration contient du charbon actif.

8. Système de filtration de l'air ambiant selon l'une quelconque des revendications précédentes, **caractérisé en ce qu'**un filtre à particules est connecté en amont de la 1^{ère} unité de filtration.

9. Utilisation d'un système de filtration de l'air ambiant selon l'une quelconque des revendications 1 à 8 pour la purification de l'air ambiant de substances gazeuses odorantes et nocives.

10. Utilisation selon la revendication 9 pour la purification de l'air ambiant de nicotine et d'autres produits issus de la combustion/carbonisation de produits de tabac.
